(19)

Europäisches
Patentamt
European
Patent Office
Office européen
des brevets

(11) **EP 3 192 879 A1**

(12) **EUROPEAN PATENT APPLICATION**
published in accordance with Art. 153(4) EPC

(43) Date of publication:
**19.07.2017 Bulletin 2017/29**

(51) Int Cl.:
***C12Q 1/68*** (2006.01)     ***C12N 15/09*** (2006.01)

(21) Application number: **15840802.1**

(22) Date of filing: **28.04.2015**

(86) International application number:
**PCT/JP2015/062893**

(87) International publication number:
**WO 2016/038929 (17.03.2016 Gazette 2016/11)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA**

(30) Priority: **11.09.2014 JP 2014185060**

(71) Applicant: **Fujifilm Corporation
Minato-ku
Tokyo 106-8620 (JP)**

(72) Inventors:
• **INOUE, Yuki
  Kanagawa 258-8538 (JP)**
• **ISHII, Yasuyuki
  Kanagawa 258-8538 (JP)**
• **OUCHI, Aya
  Kanagawa 258-8538 (JP)**

(74) Representative: **Klunker IP
Patentanwälte PartG mbB
Destouchesstraße 68
80796 München (DE)**

(54) **METHOD FOR DETECTING PRESENCE/ABSENCE OF FETAL CHROMOSOMAL ANEUPLOIDY**

(57)     Provided is a method for detecting the presence or absence of heteroploidy of fetal chromosomes, the method comprising: a primary amplification step of obtaining a primary amplification products by amplifying chromosomal DNA obtained from a biological sample collected from a pregnant mother; a secondary amplification step of performing multiplex amplification of a plurality of target regions to obtain a secondary amplification product using the primary amplification product as a template; a step of adding a label to both terminals of the secondary amplification product to obtain the labeled secondary amplification product; a tertiary amplification step of performing amplification using a primer pair annealing to the label using the labeled secondary amplification product as a template to obtain a tertiary amplification product; and a step of determining the amplification amount and base sequences of the plurality of target regions from the tertiary amplification product, in which the primary amplification step amplifies the total amount of DNA by 6000 times to 30000 times, and the secondary amplification step amplifies the total amount of DNA by 3 times to 150 times.

SEQUENCE LISTING

<110> FUJIFILM CORPORATION

<120> Method for Detection of Presence or Absence of Fetal Chromosomal Aneuploidy

<130> FS-F06249-00

<150> JP 2014-185060
<151> 2014-09-11

<160> 2

<170> PatentIn version 3.4

<210> 1
<211> 17
<212> DNA
<213> Artificial Sequence

<220>
<223> label on forward primer

<400> 1
cgctcttccg atctctg          17

<210> 2
<211> 17
<212> DNA

<213> Artificial Sequence

<220>
<223> label on reverse primer

<400> 2
cgctcttccg atctgac          17

**Description**

**BACKGROUND OF THE INVENTION**

1. Field of the Invention

The present invention relates to a method for detecting the presence or absence of heteroploidy of fetal chromosomes.

2. Description of the Related Art

In the related art, an amniotic fluid chromosome test of checking chromosomes in fetus-derived cells which have been isolated from an amniotic fluid collected through amniocentesis is performed as a prenatal genetic test. However, in amniocentesis, there is a risk of causing miscarriage.

In contrast, a test of detecting an abnormality of fetal chromosomes after analyzing fetus-derived cell-free DNA existing in blood of a pregnant mother or chromosomal DNA of a fetus-derived cell existing in blood of a pregnant mother is known as a non-invasive prenatal genetic test.

In the non-invasive prenatal genetic test, a test of analyzing fetus-derived cell-free DNA has already been put into practical use. In contrast, a test of analyzing chromosomal DNA of a fetus-derived cell has not yet been put into practical use. An example of a reason thereof is that the fetus-derived cell (for example, a fetus-derived nucleated red blood cell) is a rare cell, only about one of which exists in several mls of maternal blood. However, if it is possible to analyze chromosomal DNA of a fetus-derived cell, it is possible to expect non-invasive prenatal genetic test which is more excellent in test accuracy.

However, in order to analyze chromosomal DNA of a fetus-derived cell, it is necessary to amplify DNA from a small amount of chromosomal DNA for the purpose of obtaining sufficient amount of DNA for the analysis. At this time, it is necessary to amplify DNA while faithfully reproducing heteroploidy (for example, trisomy or monosomy) in order to detect heteroploidy of chromosomes.

A method for diagnosing a disease or parentage after obtaining sequence information from an amplification product which has been obtained by performing multi-stage DNA amplification after collecting DNA from human peripheral blood is disclosed in, for example, JP4404553B, JP2014-502845A, JP2004-511215A, and JP1994-327476A (JP-H6-327476A). In addition, a method for quantitatively determining an initial proportion of the amount of a plurality of nucleic acids by performing a multiplex amplification reaction is disclosed in JP2006-505268A.

**SUMMARY OF THE INVENTION**

As disclosed in JP4404553B, JP2014-502845A, JP2004-511215A, JP1994-327476A (JP-H6-327476A), and JP2006-505268A, a technology of analyzing sequence information or the proportion of the amount of DNA after amplifying DNA slightly existing in a sample has been known. However, the technologies disclosed in the above-described documents are not technologies which have been made from the viewpoint of improving an ability of detecting heteroploidy of chromosomes. In addition, in all of the above-described documents, there is a disclosure relating to the number of cycles of a polymerase chain reaction of amplifying DNA. However, there is no disclosure relating to a magnification of the amount of DNA before and after amplification.

One embodiment of the present invention has been made based on the above-described circumstance.

An object of the one embodiment of the present invention is to provide a method for detecting the presence or absence of heteroploidy of fetal chromosomes which has an excellent detection ability.

Specific means for solving the above-described problem include the following aspect.

[1] A method for detecting the presence or absence of heteroploidy of fetal chromosomes, the method comprising: a primary amplification step of obtaining a primary amplification products by amplifying chromosomal DNA obtained from a biological sample collected from a pregnant mother; a secondary amplification step of performing multiplex amplification of a plurality of target regions using a plurality of primer pairs to obtain a secondary amplification product using the primary amplification product as a template; a first addition step of adding a first label, which is an oligonucleotide, to both terminals of the secondary amplification product to obtain the labeled secondary amplification product; a tertiary amplification step of performing amplification using a primer pair annealing to the first label using the labeled secondary amplification product as a template to obtain a tertiary amplification product; and a sequence analysis step of determining the amplification amount and base sequences of the plurality of target regions from the tertiary amplification product, in which the primary amplification step is a step of amplifying the total amount of DNA by 6000 times to 30000 times, and the secondary amplification step is a step of amplifying the total amount of DNA by 3 times to 150 times.

[2] The method for detecting the presence or absence of heteroploidy of fetal chromosomes according to [1], in which the tertiary amplification step is a step of amplifying the total amount of DNA by 6 times to 24 times.

[3] The method for detecting the presence or absence of heteroploidy of fetal chromosomes according to [1] or [2], in which the sequence analysis step is performed using a next-generation sequencer.

[4] The method for detecting the presence or absence of heteroploidy of fetal chromosomes according to any one of [1] to [3], the method further comprising: a second addition step of adding a second label, which is an oligonucleotide, to both terminals of the tertiary amplification product to obtain the labeled tertiary amplification product.

[5] The method for detecting the presence or absence of heteroploidy of fetal chromosomes according to [4], in which the primer used in the tertiary amplification step has a base sequence of the second label at the 5'-terminal, and the second label is added to both terminals of the tertiary amplification product and the labeled tertiary amplification product is obtained by performing the tertiary amplification step which is performed together with the second addition step in this manner.

[6] The method for detecting the presence or absence of heteroploidy of fetal chromosomes according to any one of [1] to [5], in which the primer used in the secondary amplification step has a base sequence of the first label at the 5'-terminal, and the first label is added to both terminals of the secondary amplification product and the labeled secondary amplification product is obtained by performing the secondary amplification step which is performed together with the first addition step in this manner.

[7] The method for detecting the presence or absence of heteroploidy of fetal chromosomes according to any one of [1] to [6], in which the primary amplification step is a step of performing whole genome amplification from the chromosomal DNA.

[8] The method for detecting the presence or absence of heteroploidy of fetal chromosomes according to any one of [1] to [7], in which the chromosomal DNA is chromosomal DNA obtained from one cell, and the primary amplification step is a step of performing whole genome amplification from one cell.

[9] The method for detecting the presence or absence of heteroploidy of fetal chromosomes according to any one of [1] to [8], in which the biological sample is blood, and the chromosomal DNA is chromosomal DNA obtained from a cell in blood.

[10] The method for detecting the presence or absence of heteroploidy of fetal chromosomes according to [9], in which the cell in blood is a nucleated red blood cell.

[11] The method for detecting the presence or absence of heteroploidy of fetal chromosomes according to [9] or [10], in which the cell in blood is a cell isolated through density gradient centrifugation and image analysis.

According to the one embodiment of the present invention, there is provided a method for detecting the presence or absence of heteroploidy of fetal chromosomes which has an excellent detection ability.

**DESCRIPTION OF THE PREFERRED EMBODIMENTS**

The term "step" in the present specification does not only refer to an independent step, and other steps in a case where there is no clear distinction between the step and the other steps are also included in this term as long as expected purposes of the steps are achieved.

A numerical range shown by "to" in the present specification indicates a range including numerical values described before and after "to" as minimum values and maximum values.

Unless otherwise specified, the amount of each component in a composition in the present specification means a total amount of a plurality of substances existing in the composition in a case where the plurality of substances corresponding to each components exist in the composition.

Hereinafter, the embodiment of the present invention will be described. These description and Examples exemplify the present invention and do not limit the scope of the present invention.

The method of the present disclosure is a method for detecting the presence or absence of heteroploidy of fetal chromosomes in which chromosomal DNA is obtained from a biological sample collected from a pregnant mother and a plurality of regions on the chromosomal DNA are amplified. The method of the present disclosure detects the presence or absence of heteroploidy of fetal chromosomes after amplifying DNA in multi-stages and determining the amount of the amplification product and a base sequence of the amplification product, through a primary amplification step, a secondary amplification step, and a tertiary amplification step. The method of the present disclosure is applied to a prenatal genetic test of detecting, for example, trisomy of chromosome 13, chromosome 18, and chromosome 21; and surplus of sex chromosomes.

In the method of the present disclosure, the primary amplification step, the secondary amplification step, and the tertiary amplification step amplify DNA using DNA polymerase. The primary amplification step is a step of amplifying the total amount of DNA by 6000 times to 30000 times and the secondary amplification step is a step of amplifying the total amount of DNA by 3 times to 150 times.

The method of the present disclosure is excellent in an ability of detecting the presence or absence of heteroploidy of fetal chromosomes by setting an amplification factor (total amount of DNA after amplification / total amount of DNA before amplification) of the total amount of DNA to be within the above-described range in the primary amplification step and the secondary amplification step. The reason for that is not restricted by a specific mechanism. However, it is assumed that the reason is based on a mechanism described below.

In DNA amplification performed using DNA polymerase, easiness of amplification (amplification efficiency) varies in each amplification region depending on a base sequence of an amplification region, the length of an amplification region, the uniqueness of a primer sequence, the reaction temperature, the reaction time, or the like, and the differences in the amplification efficiency accumulate every time amplification cycles are repeated. As a result, in some cases, the proportion of the amount of amplification products between amplification regions does not reproduce the proportion of the original amount of DNA.

In addition, a polymerase chain reaction (PCR) which is one of DNA amplification methods is theoretically a reaction of exponentially amplifying DNA by repeating cycles. However, in reality, the concentration of DNA (that is, the amount of amplification product) reaches a plateau in a case where cycles are repeated. For this reason, in a case where the number of cycles is excessive, the amounts of amplification products become almost the same as each other even if there is a difference with the original amount of DNA. Therefore, the information of the proportion of the original amount of DNA is lost.

Regarding this, the amplification factor of the total amount of DNA in the primary amplification step is set to less than or equal to 30000 times in the method of the present disclosure. In a case where the amplification factor exceeds 30000 times, a certain number of amplification cycles are correspondingly required in order to realize this amplification factor, and the differences in the amplification efficiency accumulate every time the amplification cycles are repeated. As a result, the proportion of the amount of amplification products between amplification regions differs from the proportion of the original amount of DNA in chromosomes. Therefore, in the method of the present disclosure, the amplification factor of the total amount of DNA in the primary amplification step is less than or equal to 30000 times. In a case where the amplification factor of the total amount of DNA in the primary amplification step is less than or equal to 30000 times, amplification factors in each amplification region amplified through the primary amplification step also fall within a range of less than or equal to about 30000 times. Therefore, variation in distribution of the amplification factors is reduced and the information of heteroploidy of chromosomes is maintained. For example, in a case where the amplification factor of the total amount of DNA is 10000 times, the amplification factors in each amplification region are distributed in the vicinity of 10000 times, and therefore, the variation is small.

It can be said that the amplification factor of the total amount of DNA in the secondary amplification step also has the same mechanism as that described above. Therefore, in the method of the present disclosure, the amplification factor of the total amount of DNA in the secondary amplification step is less than or equal to 150 times. In addition, the secondary amplification step is usually performed through PCR. Therefore, even from the viewpoint of avoiding the information of heteroploidy of chromosomes from being lost through excessive amplification, the number of amplification cycles may not be too excessive and the amplification factor of the total amount of DNA is less than or equal to 150 times. In a case where the amplification factor of the total amount of DNA in the secondary amplification step is less than or equal to 150 times, the amplification factors of each amplification region amplified through the secondary amplification step also fall within a range of less than or equal to about 150 times. Therefore, variation in distribution of the amplification factors is reduced and the information of heteroploidy of chromosomes is maintained. For example, in a case where the amplification factor of the total amount of DNA is 100 times, the amplification factors in each amplification region are distributed in the vicinity of 100 times, and therefore, the variation is small.

In contrast, it is necessary to obtain an amount of DNA sufficient for analysis, from a small amount of chromosomal DNA in a biological sample. Therefore, the amplification factor of the total amount of DNA in the primary amplification step is greater than or equal to 6000 times and the amplification factor of the total amount of DNA in the secondary amplification step is greater than or equal to 3 times.

Due to the above-described reason, the primary amplification step is a step of amplifying the total amount of DNA by 6000 times to 30000 times, more preferably a step of amplifying the total amount of DNA by 6000 times to 25000 times, and still more preferably a step of amplifying the total amount of DNA by 6000 times to 20000 times.

Due to the above-described reason, the secondary amplification step is a step of amplifying the total amount of DNA by 3 times to 150 times, more preferably a step of amplifying the total amount of DNA by 3 times to 100 times, and still more preferably a step of amplifying the total amount of DNA by 3 times to 60 times.

In the method of the present disclosure, the amplification factor of the total amount of DNA in the tertiary amplification step is not particularly limited. In the tertiary amplification step, the size of DNA which becomes a template is aligned and a primer pair to be used is usually limited to one kind. Therefore, it is considered that the problem relating to DNA amplification is not manifested in the primary amplification step and the secondary amplification step. However, the amplification factor of the total amount of DNA in the tertiary amplification step is preferably 6 times to 24 times, more preferably 6 times to 20 times, and still more preferably 6 times to 15 times due to the same reason and mechanism as those in the secondary amplification step. In a case where the amplification factor of the total amount of DNA in the tertiary amplification step is within the above-described range, the method of the present disclosure becomes more excellent in an ability of detecting the presence or absence of heteroploidy of fetal chromosomes.

The whole or a part of a final amplification product, that is, a tertiary amplification product which has been amplified through the above-described primary amplification step, secondary amplification step, and tertiary amplification step in multi-stages is a substance fed in a device for sequence analysis in the sequence analysis step. The tertiary amplification product is, so to speak, a library for a sequence. In the method of the present disclosure, the proportion between the amounts of tertiary amplification products of a plurality of target regions well reproduces the proportion between the original amounts of the DNAs (that is, heteroploidy of chromosomes) in chromosomal DNA. Accordingly, it is possible to accurately detect the presence or absence of heteroploidy of chromosomes according to the method of the present disclosure.

Hereinafter, each step will be described in detail.

[Primary Amplification Step]

The primary amplification step is a step of obtaining a primary amplification product after amplifying chromosomal DNA obtained from a biological sample collected from a pregnant mother. The primary amplification step may be a synthesis reaction of DNA chains which is performed by DNA polymerase. The biological sample collected from a pregnant mother will be described below.

Primary amplification is preferably performed through whole genome amplification (WGA). In the whole genome amplification, it is possible to amplify, for example, a small amount of a chromosomal DNA fragment contained in a biological sample or a small amount of chromosomal DNA obtained from a single cell, and therefore, analysis of chromosomal DNA is facilitated.

The whole genome amplification method is not particularly limited, and may be performed through a well-known method. The whole genome amplification includes a step of dissolving cells using a surfactant and a proteolytic enzyme, and a step of amplifying DNA using DNA polymerase and using genome DNA, eluted from cells, as a template.

The whole genome amplification may be performed by applying a commercially available reagent. Examples of reagents based on PCR include PicoPLEX WGA Kit (New England Biolabs), GenomePlex Single Cell Whole Genome Amplification kit (Sigma-Aldrich Co. LLC.), and reagents relating to a Multiple Annealing and Looping-Based Amplification Cycles (MALBAC) method disclosed in WO2012/166425A. In addition, examples of reagents based on a chain-substituting DNA synthesis reaction include GenomiPhi DNA Amplification Kit (GE Healthcare, GenomiPhi is a registered trademark) and REPLI-g Single Cell Kit (QIAGEN, REPLI-g is a registered trademark). In the method of the present disclosure, it is preferable to use PicoPLEX WGA Kit (New England Biolabs).

The PicoPLEX WGA Kit (New England Biolabs) is a kit used for sequentially performing three steps including (i) Sample Preparation, (ii) DNA Pre-Amplification, and (iii) DNA Amplification, in one reaction container.

Step (i) is a step of extracting genome DNA from one cell, in which a cell is dissolved using a buffer containing a surfactant and a proteolytic enzyme.

Step (ii) is a step of pre-amplifying DNA using genome DNA extracted from a cell, a random primer, and heat-resistant DNA polymerase.

Step (iii) is a step of amplifying DNA after performing PCR using a heat-resistant DNA polymerase.

In a case where PicoPLEX WGA Kit (New England Biolabs) is applied to the primary amplification step, it is preferable to omit step (iii) while performing step (i) and step (ii) so as to make the total amount of DNA be amplified by 6000 times to 30000 times, and it is more preferable to control the number of amplification cycles of step (ii).

After the completion of the primary amplification step, it is preferable to check the presence or absence of a primary amplification product by performing agarose gel cataphoresis. The primary amplification product is preferably purified. The purification is performed using, for example, a QIAquick PCR Purification Kit (QIAGEN, QIAquick is a registered trademark). The amount of the primary amplification product can be checked by measuring the concentration using, for example, NanoDrop (registered trademark, Thermo Fisher Scientific Inc.), BioAnalyzer (Agilent Technologies), and Quantus Fluorometer (Promega KK.).

[Secondary Amplification Step and First Addition Step]

The secondary amplification step is a step of performing multiplex amplification of a plurality of target regions using a plurality of primer pairs using the primary amplification product as a template to obtain a secondary amplification product. The secondary amplification product is a mixture of a plurality of amplification products. The secondary amplification step is preferably realized through PCR, that is, multiplex PCR.

The primer pairs used in the secondary amplification step are primer pairs designed for the purpose of detecting heteroploidy of fetal chromosomes and are a plurality of primer pairs corresponding to a plurality of chromosomal locations (target regions). The length of the target regions, that is, the length of regions to be amplified is, for example, 100 to 180 base pairs. As a base sequence complementary to the target regions in the primers, 15 to 25 bases are preferable and 20 bases are more preferable.

The first addition step is a step of adding a first label to both terminals of the secondary amplification product to obtain the labeled secondary amplification product. The first label is an oligonucleotide and is a sequence in which a primer used in the tertiary amplification step is annealed. A base sequence in a first label added to one terminal of the secondary amplification product and a base sequence in a first label added to the other terminal may be the same as or different from each other. In the present disclosure, a label to be added to both terminals of the secondary amplification product is collectively called a "first label" regardless of the difference in the base sequences of both labels.

The first label is a sequence in which a primer used in the tertiary amplification step is annealed. Therefore, by sharing the first label between secondary amplification products, it is possible to perform PCR using one type of primer pair in the tertiary amplification step. In the first label, 15 to 20 bases are preferable and 17 bases are more preferable.

Examples of the method for adding a first label to both terminals of the secondary amplification product include ligation performed using DNA ligase and addition performed through PCR. In the method of the present disclosure, addition performed through PCR is preferable. That is, the addition performed through PCR is obtaining of a secondary amplification product obtained by adding a first label to both terminals by performing PCR using a primer having a base sequence of the first label at the 5'-terminal. In this case, the secondary amplification step and the first addition step are performed together.

In a case where the primer used in the secondary amplification step has a base sequence of the first label and a base sequence complementary to a target region, the total length of the primer is preferably 30 to 45 bases, more preferably 35 to 40 bases, and still more preferably 37 bases.

It is possible to use heat-resistant DNA polymerase and a reaction buffer, which are used for usual PCR, for the multiplex PCR of the secondary amplification step. Examples of PCR reagents applied to the secondary amplification step include Multiplex PCR Assay Kit (TAKARA BIO INC.), Multiplex PCR Assay Kit ver2 (TAKARA BIO INC.), KAPA Library Amplification Kit (NIPPON Genetics Co, Ltd), and Platinum Multiplex PCR Master Mix Kit (Life Technologies Corporation, Platinum is a registered trademark). In the method of the present disclosure, it is preferable to use Multiplex PCR Assay Kit (TAKARA BIO INC.). In the multiplex PCR, optimum temperatures for annealing are different in each primer pair, and therefore, it is preferable to check reaction conditions.

In the method of the present disclosure for detecting the presence or absence of heteroploidy of chromosomes, it is preferable to set the number of cycles of PCR of the secondary amplification step to 10 to 30 cycles in order to prevent the information of heteroploidy of chromosomes from being lost due to excessive amplification. It is preferable that the number of cycles of PCR is set based on results obtained by performing review through real time PCR in advance. In PCR, the reaction temperature and/or the reaction time may be changed in the middle of the process.

It is preferable to purify the secondary amplification product. The purification is performed using, for example, QIAquick PCR Purification Kit (QIAGEN) and AMPure XP Kit (BECMAN COULTER). The amount of the secondary amplification product can be checked by measuring the concentration using, for example, NanoDrop (Thermo Fisher Scientific Inc.), BioAnalyzer (Agilent Technologies), and Quantus Fluorometer (Promega KK.).

[Tertiary Amplification Step and Second Addition Step]

The tertiary amplification step is a step of performing amplification using a primer pair annealing to the first label using the labeled secondary amplification product as a template to obtain a tertiary amplification product. The tertiary amplification step is preferably realized through PCR.

The primer used for the tertiary amplification step is a primer annealing to the first label. It is preferable that the base length of a sequence annealing to the first label in the primer is the same as that of the first label. Specifically, 15 to 20 bases are preferable and 17 bases are more preferable.

The second addition step is a step of adding a second label to both terminals of the tertiary amplification product to obtain the labeled tertiary amplification product. The second label is an oligonucleotide and is added to the tertiary amplification product for the purpose of performing analysis of the amplification product using a next-generation sequencer. A base sequence in a second label added to one terminal of the tertiary amplification product and a base sequence in a second label added to the other terminal may be the same as or different from each other. In the present disclosure, a label to be added to both terminals of the tertiary amplification product is collectively called a "second label" regardless of the difference in the base sequences of both labels.

Since the second label is an oligonucleotide added to a tertiary amplification product for the purpose of performing analysis of the amplification product using a next-generation sequencer, the base sequence thereof is designed in accordance with an analysis principle of the next-generation sequencer (the details of the next-generation sequencer will be described below). Examples of the base length of the second label include 59 to 64 bases.

In a case of using MiSeq or HiSeq 2000 of Illumina, Inc. as a next-generation sequencer, examples of the second label include a pair of oligonucleotides described below. One out of the two oligonucleotides described below is added to one terminal of a tertiary amplification product and the other oligonucleotide is added to the other terminal of the tertiary amplification product.

- An oligonucleotide having a P5 sequence hybridizing to an oligonucleotide fixed to the top of a flow cell, a sample identification sequence (index sequence) formed of 6 to 8 bases, and a sequence (lead sequence) in which a sequence primer is annealed.
- An oligonucleotide having a P7 sequence hybridizing to an oligonucleotide fixed to the top of a flow cell, a sample identification sequence (index sequence) formed of 6 to 8 bases, and a sequence (lead sequence) in which a sequence primer is annealed.

The index sequence may be at least one of the two oligonucleotides becoming a pair, and is preferably both of the two oligonucleotides from the viewpoint of increasing the analysis accuracy. The first label can be allocated to a lead sequence, and in this case, the lead sequence may not be separately installed.

The above-described example is an example, and the second label is not limited to the above-described example in the method of the present disclosure.

Examples of the method for adding the second label to both terminals of the tertiary amplification product include ligation performed using DNA ligase and addition performed through PCR. In the method of the present disclosure, addition performed through PCR is preferable. That is, the addition performed through PCR is obtaining of a tertiary amplification product obtained by adding a second label to both terminals by performing PCR using a primer having a base sequence of the second label at the 5'-terminal. In this case, the tertiary amplification step and the second addition step are performed together.

Hereinafter, in some cases, the addition of the second label through PCR is called "index addition PCR".

Examples of PCR reagents applied to the tertiary amplification step include Multiplex PCR Assay Kit (TAKARA BIO INC.), Multiplex PCR Assay Kit ver2 (TAKARA BIO INC.), KAPA Library Amplification Kit (NIPPON Genetics Co, Ltd), and Platinum Multiplex PCR Master Mix Kit (Life Technologies Corporation). In the method of the present disclosure, it is preferable to use Multiplex PCR Assay Kit (TAKARA BIO INC.).

In the method of the present disclosure for detecting the presence or absence of heteroploidy of chromosomes, it is preferable to set the number of cycles of PCR of the tertiary amplification step to 5 to 18 cycles in order to prevent the information of heteroploidy of chromosomes from being lost due to excessive amplification. It is preferable that the number of cycles of PCR is set based on results obtained by performing review through real time PCR in advance. In PCR, the reaction temperature and/or the reaction time may be changed in the middle of the process.

It is preferable to purify the tertiary amplification product. The purification is performed using, for example, QIAquick PCR Purification Kit (QIAGEN) and AMPure XP Kit (BECMAN COULTER). The amount of the tertiary amplification product can be checked by measuring the concentration using, for example, NanoDrop (Thermo Fisher Scientific Inc.), BioAnalyzer (Agilent Technologies), Quantus Fluorometer (Promega KK.), and KAPA Library Quantification Kits (NIPPON Genetics Co, Ltd).

[Sequence Analysis Step]

The sequence analysis step is a step of determining the amplification amount and base sequences of a plurality of target regions from the tertiary amplification product. It is preferable that the sequence analysis step is performed using a next-generation sequencer in terms of the accuracy and the speed of analysis, a large number of samples which can be treated at a time, or the like.

In the present disclosure, the next-generation sequencer (NGS) means a sequencer which is classified in contrast to a capillary sequencer (called a first generation sequencer) in which a Sanger method is used. The next-generation sequencer includes a second generation sequencer, a third generation sequencer, a fourth generation sequencer, and a sequencer to be developed later. The most common next-generation sequencer at this point in time is a sequencer which has a principle of determining a base sequence after grasping fluorescence or light emission and is linked with complementary strand synthesis performed using DNA polymerase or complementary strand binding performed using DNA ligase. Specific examples thereof include MiSeq (Illumina, Inc.), HiSeq 2000 (Illumina, Inc., HiSeq is a registered trademark), and Roche 454 (Roche Diagnostics K.K.).

In the method of the present disclosure, MiSeq and HiSeq 2000 of Illumina, Inc. are suitable as next-generation sequencers. MiSeq can measure up to 96 types of PCR products at a time. The 96 types of PCR products may include one multiplex PCR product or a mixture of a plurality of multiplex PCR products. In a case of performing analysis using MiSeq after mixing the plurality of multiplex PCR products, it is desirable to accurately quantitatively determine each of the PCR products. Quantitative determination is performed using, for example, BioAnalyzer (Agilent Technologies), Quantus Fluorometer (Promega KK.), or KAPA Library Quantification Kits (NIPPON Genetics Co, Ltd).

Examples of means for aligning sequence data pieces obtained through a next-generation sequencer such as MiSeq include Burrows-Wheeler Aligner (BWA), and it is preferable to map the sequence data pieces in well-known human genome sequences using BWA. Examples of means for analyzing genes include SAMtools and BEDtools, and it is preferable to analyze genetic polymorphism, genetic mutation, and the number of chromosomes using these analysis means.

The presence or absence of heteroploidy of chromosomes is detected from the amplification amount and base sequences of target regions which have been obtained in the sequence analysis step. The details of the sequence analysis step and the method for detecting heteroploidy will be described below while having a case, in which nucleated red blood cells in maternal blood are set as samples, as an example.

[Biological Sample Collected from Pregnant Mother]

Biological samples collected from a pregnant mother may be maternal blood, cord blood, an amniotic fluid, and tissue, such as villus tissue and placental tissue, which is known that there are fetus-derived cells. Maternal peripheral blood is preferably as a biological sample from the viewpoint of suppressing invasiveness to a pregnant mother as much as possible. Blood itself and blood samples such as blood diluted with a physiological salt solution; preserved blood obtained by adding an additive such as glucose or an anticoagulant to blood; and a fractionated product thereof are included in blood in the present disclosure.

[Peripheral Blood of Pregnant Mother]

Blood cells such as: mother-derived leucocytes such as eosinophils, neutrophils, basophils, monocytes, and lymphocytes; mother-derived mature red blood cells having no nucleus; mother-derived nucleated red blood cells; and fetus-derived nucleated red blood cells are included in peripheral blood of a pregnant mother. In the method of the present disclosure, it is preferable to obtain chromosomal DNA from fetus-derived nucleated red blood cells which are identified from these blood cells. It is known that the fetus-derived nucleated red blood cells exist in maternal blood after about 6 weeks of pregnancy. Accordingly, it is preferable that blood in the method of the present disclosure is peripheral blood collected from a pregnant mother after about 6 weeks of pregnancy or a blood sample prepared from peripheral blood collected from a pregnant mother after about 6 weeks of pregnancy.

Fetus-derived nucleated red blood cells are erythrocyte precursors which exist in blood of a mother after passing through the placenta. Red blood cells of a fetus can be nucleated during pregnancy of a mother. Since there are chromosomes in these nucleated red blood cells, it is possible to obtain fetal chromosomes and fetal genes through isolation of fetus-derived nucleated red blood cells. It is known that these fetus-derived nucleated red blood cells exist at a ratio of one fetus-derived nucleated red blood cell to about $10^6$ cells in maternal blood. Therefore, the existence probability of the fetus-derived nucleated red blood cells in peripheral blood of a pregnant mother is significantly low.

Fetus-derived nucleated red blood cells can be isolated by being identified from other blood cells existing in blood collected from a pregnant mother through, for example, density gradient centrifugation and image analysis.

[Density Gradient Centrifugation of Nucleated Red Blood Cell (Concentration of Nucleated Red Blood Cell)]

Nucleated red blood cells can be separated from plasma components or other blood cells existing in blood, through density gradient centrifugation. Any well-known method may be applied for the density gradient centrifugation for performing separation of nucleated red blood cells. For example, nucleated red blood cells can be fractionated and concentrated by performing centrifugation after making blood, which has been diluted with a physiological salt solution, overlap the top of a discontinuous density gradient which has been obtained by making two types of media having different densities (specific gravities) from each other overlap each other in a centrifuge tube.

The density of blood cells of a mother containing fetus-derived nucleated red blood cells is disclosed in WO2012/023298A. According to the disclosure, the density of fetus-derived nucleated red blood cells which is assumed is about 1.065 to 1.095 g/mL, the density of blood cells of a mother is about 1.070 to 1.120 g/mL in a case of red blood cells, about 1.090 to 1.110 g/mL in a case of eosinophils, about 1.075 to 1.100 g/mL in a case of neutrophils, about 1.070 to 1.080 g/mL in a case of basophils, about 1.060 to 1.080 g/mL in a case of lymphocytes, and about 1.060 to 1.070 g/mL in a case of monocytes.

The density (specific gravity) of media to be stacked is set in order to separate fetus-derived nucleated red blood cells having a density of about 1.065 to 1.095 g/mL from other blood cells. The density of the center of fetus-derived nucleated red blood cells is about 1.080 g/mL. Therefore, it is possible to collect fractions having fetus-derived nucleated red blood cells on an interface between two adjacent media having different densities interposing the density by making the media overlap each other. The density of the lower medium is preferably 1.08 g/mL to 1.10 g/mL (more preferably 1.08 g/mL to 1.09 g/mL), the density of the upper medium is preferably 1.06 g/mL to 1.08 g/mL (more preferably 1.065 g/mL to 1.08 g/mL). In the method of the present disclosure, the type of the lower medium and the type of the upper medium may be the same as or different from each other. It is preferable to use the same types of media.

Examples of the media include Percoll (registered trademark) which is a silica colloid particle dispersion liquid which has a diameter of 15 nm to 30 nm and is coated with polyvinyl pyrrolidone, Ficoll-Paque (registered trademark) which is a neutral hydrophilic polymer rich in a side chain made of sucrose, and Histopaque (registered trademark) which contains polysucrose and sodium diatrizoate. In the method of the present disclosure, it is preferable to use Percoll and/or Histopaque. A product of Percoll having a density of 1.130 is commercially available and it is possible to prepare a density gradient by diluting the product with water. It is possible to prepare density gradients using water and a medium having a density of 1.077 and a medium having a density of 1.119 which are commercially available as Histopaque.

The double-layered discontinuous density gradients are formed in, for example, a centrifuge tube as described below. First, the lower medium, which is in a state of a temperature of higher than or equal to a solidifying point and lower than or equal to 14°C (preferably lower than or equal to 8°C), is stored in the bottom portion of the centrifuge tube, or is cooled while being preserved under a temperature of lower than or equal to 14°C (preferably lower than or equal to 8°C) after being stored in the bottom portion of the centrifuge tube. Next, the upper medium is made to overlap the top of the lower medium.

[Image Analysis of Blood Cell]

Image analysis of blood cells is performed after obtaining information of the forms of blood cells on a substrate (preferably a transparent substrate) for the purpose of selecting a candidate of a fetus-derived nucleated red blood cell.

In order to perform image analysis of blood cells, a specimen for image analysis is produced after coating the top of a transparent substrate (preferably, slide glass) with fractions which contain nucleated red blood cells obtained through density gradient centrifugation and are then dried. A candidate of a fetus-derived nucleated red blood cell is selected after analyzing the information of the shapes of the blood cells obtained from this specimen.

The blood cells of the specimen for image analysis are preferably dyed in order to easily observe the blood cells. The method for dyeing blood cells is not particularly limited, and any well-known method may be performed. Examples of the method for dyeing blood cells include GIEMSA dyeing and MAY-Grunwald dyeing.

Image analysis is preferably performed on the information of the shapes of blood cells which has been obtained from a specimen using a system equipped with an optical microscope, a digital camera, a stage for slide glass, an optical transfer system, an image processing personal computer (PC), a control PC, and a display. The optical transfer system includes, for example, an objective lens and a CCD camera. The image processing PC include, for example, a processing system of performing data analysis and storing of data. The control PC includes, for example, a control system of controlling the position of a stage for slide glass or controlling the entire processing.

It is possible to identify a candidate of a fetus-derived nucleated red blood cell in accordance with the ratio of the area of a nucleus to the area of cytoplasm, the roundness of a nucleus, the area of a nucleus, or the like. It is preferable to identify a cell, which satisfies at least one condition (preferably both conditions) of the ratio of the area of a nucleus to the area of cytoplasm and the roundness of a nucleus, as a fetus-derived nucleated red blood cell candidate.

Regarding the ratio of the area of a nucleus to the area of cytoplasm, it is preferable to select a cell satisfying the following Formula (a). Regarding the roundness of a nucleus, it is preferable to select a cell satisfying the following Formula (b).

$$(a)\ 0.25 < (N / C) < 1.0$$

$$(b)\ 0.65 < (N / (L \times L)) < 0.785$$

In Formulas, C represents the area of cytoplasm, N represents the area of a nucleus, L represents the length of a major axis of a nucleus or the length of a major axis of an ellipse circumscribing a nucleus which has a complicated shape.

Prioritization may be performed on cells satisfying Formula (a) and Formula (b), in order in which the shape of a nucleus is close to a perfect circle or an ellipse, and cells with a high priority may be preferentially provided in the following step. In addition, in a case where there is no fetus-derived cell in a plurality of cells having a high priority after performing the process up to a step of discriminating the origin of nucleated red blood cells, a plurality of cells having a subsequently high priority may be analyzed.

Cells identified as nucleated red blood cells are collected one by one from a transparent substrate using, for example, a micromanipulator including a glass instrument. Chromosomal DNA is extracted from the collected cells and becomes a template of a primary amplification step.

[Identification of Fetus-Derived Nucleated Red Blood Cell]

Mother-derived nucleated red blood cells and fetus-derived nucleated red blood cells are mixed with each other in nucleated red blood cells in blood collected from a pregnant mother, and therefore, it is necessary to identify fetus-derived nucleated red blood cells. Examples of a method for identifying an individual using a genetic sequence include a method for checking an allele and detecting genetic polymorphism existing in the allele. For example, a method for detecting short tandem repeat (STR) which is one type of genetic polymorphism is applied in a case of discriminating a father-son relation and a method for detecting single nucleotide polymorphism (SNP, single base polymorphism) in a case of identifying an individual. In the method of the present disclosure, fetus-derived cells and mother-derived cells are identified in accordance with, for example, the difference between STR and/or SNP on an allele based on sequence information obtained in the sequence analysis step. It is preferable to improve reliability of identifying fetus-derived cells by obtaining white blood cells (white blood cells are almost certainly derived from a mother) and analyzing STR and/or SNP in the same manner.

[Y Chromosome Detection in Case of Male Fetus]

In a case where it is confirmed that a fetus is a male fetus in an ultrasonic test of a pregnant mother, if it is confirmed that there is a Y chromosome within an isolated nucleated red blood cell, it is possible to identify that the nucleated red blood cell is a fetus-derived nucleated red blood cell. A fluorescence in situ hybridization (FISH) method in which Y chromosome-specific fluorescent probe is known as the method for detecting the presence or absence of a Y chromosome within a cell. Examples of a test kit of the FISH method include CEP X/Y DNA Probe Kit (Abbott, CEP is a registered trademark). In the method of the present disclosure, it is preferable to identify a fetus-derived nucleated red blood cell of a male fetus by checking the presence or absence of amplification of a primer pair, which has been produced and has a base sequence specific to a Y chromosome, using PCR.

[Detection of Presence or Absence of Heteroploidy of Chromosome]

The amount of amplification product of a chromosome obtained from a cell which has been identified as a fetus-derived nucleated red blood cell is analyzed using, for example, a next-generation sequencer. As a standard (or a reference), the amount of amplification product of a chromosome obtained from a cell which has been identified as a mother-derived nucleated red blood cell is analyzed using, for example, a next-generation sequencer. In a case where a fetus does not have heteroploidy of a chromosome, it is expected that the ratio of the amount of amplification product derived from a mother to the amount of amplification product derived from a fetus becomes almost 1:1. In a case where a chromosome at which an amplification region is positioned is trisomy in a fetus, it is expected that the ratio of the amount of amplification product derived from a mother to the amount of amplification product derived from a fetus becomes almost 1:1.5 (or 2:3).

In the method of the present disclosure, a cutoff value may be determined in advance through the following method and may be used for interpreting an analysis result.

From biological samples collected from a plurality of mothers who are determined to be pregnant with fetuses having no heteroploidy of chromosomes, the proportions of the amounts of amplification products derived from fetuses to the amounts of amplification products derived from mothers are analyzed through the method of the present disclosure, and distributions thereof are obtained. In addition, from biological samples collected from a plurality of mothers who are determined to be pregnant with fetuses with trisomy, the proportions of the amounts of amplification products derived from fetuses to the amounts of amplification products derived from mothers are analyzed through the method of the present disclosure, and distributions thereof are obtained. A region in which these two distributions do not overlap is set to a cutoff value. This cutoff value is compared with the ratio of the amount of amplification product derived from a fetus to the amount of amplification product derived from a mother in a target to be tested, and it is possible to interpret that a fetus is not trisomy if the ratio thereof is less than or equal to the cutoff value and a fetus is trisomy if the ratio thereof is greater than or equal to the cutoff value.

[Examples]

The present invention will be described in more detail using examples exemplified below. Materials, processing procedures, and the like shown in the following Examples can be appropriately changed as long as they do not depart from the gist of the present invention. However, the scope of the present invention is not restrictively interpreted using specific examples shown below.

<Example 1>

[Collection of Peripheral Blood]

7 mL of peripheral blood was collected in a 7 mL blood collecting tube, to which 10.5 mg of sodium salts of ethylenediaminetetraacetic acid (EDTA) were added as an anticoagulant, from a pregnant woman volunteer. Thereafter, blood was diluted with a physiological salt solution. Collection of blood from a pregnant woman volunteer was performed after obtaining informed consent.

[Concentration of Nucleated Red Blood Cell through Density Gradient Centrifugation]

A liquid with a density of 1.070 and a liquid with a density of 1.095 were prepared using a Percoll liquid (Sigma-Aldrich Co. LLC.), 2 mL of a liquid with a density of 1.095 was added to a centrifuge tube, and the mixture was cooled while being allowed to stand for 30 minutes at lower than 4°C. Thereafter, 2 mL of a liquid with a density of 1.070 was made to slowly overlap the top of the liquid with a density of 1.095 so as not to disturb an interface. Continuously, 11 mL of diluted blood was made to slowly overlap the top of the liquid with a density of 1.070. Centrifugation was performed for 20 minutes at 2000 rpm. Next, fractions which had been deposited between the liquid with a density of 1.070 and the liquid with a density of 1.095 were collected using a pipette.

[Coating Substrate with Blood]

Slide glass 1 was held by one hand and a droplet of the collected fractions was spotted at one end of the slide glass. Slide glass 2 was held by the other hand and one end of the slide glass 2 was brought into contact with the slide glass 1 at 30 degrees. The lower contact surface of the slide glass 2 which was brought into contact with the fractions was then spread into a space surrounded by the two sheets of glass due to a capillary phenomenon. Next, the slide glass 2 was made to be slid in a direction of a region opposite to the region of the slide glass 1, on which fractions were placed, while maintaining the above-described angle, and the slide glass 1 was uniformly coated with the fractions. After the coating, the slide glass 1 was sufficiently dried through air blowing for one or more hours.

[Dyeing of Blood Cell]

The slide glass 1 which was coated with the fractions was immersed in a MAY-Grunwald dyeing liquid for three minutes, and was then washed by being immersed in a phosphoric acid buffer solution. Thereafter, the slide glass 1 was immersed in a GIEMSA dyeing liquid (which was diluted with a phosphoric acid buffer solution to make a concentration of 3% (v/v)) for 10 minutes. Next, the slide glass 1 was dried after being washed with pure water. A plurality of sheets of specimen glass for image analysis were produced in this manner.

[Identification of Nucleated Red Blood Cell Performed through Image Analysis]

An image analysis system which includes an optical microscope provided with an electric XY stage, an objective lens, and a CCD camera, a control unit provided with an XY stage control unit and a Z-direction control unit, and an analysis unit provided with an image input unit, image processing unit, and an XY position recording unit was prepared. The specimen slide was placed on the XY stage, scanning was performed by being focused on the specimen slide, an image from an optical microscope was taken in the analysis unit, and nucleated red blood cells were searched.
Cells which satisfy the following Formula (a) and Formula (b) were detected through image analysis, a nucleated red blood cell candidate was identified, and the XY positions were recorded.

$$\text{(a) } 0.25 < (N / C) < 1.0$$

$$\text{(b) } 0.65 < (N / (L \times L)) < 0.785$$

In Formulas, C represents the area of cytoplasm, N represents the area of a nucleus, L represents the length of a major axis of a nucleus or the length of a major axis of an ellipse circumscribing a nucleus which has a complicated shape.

[Collection of Nucleated Red Blood Cell]

A glass needle and a glass tube were operated using a micromanipulator, and 11 nucleated red blood cell candidates which had been identified were collected from the top of the specimen slide one by one using these glass instruments.

[Primary Amplification Step (Whole Genome Amplification)]

Whole genome amplification was performed on each of the collected 11 cells using PicoPLEX WGA Kit (New England Biolabs). After 5 μL of Cell Extraction Buffer was added to a PCR tube in which one cell is stored, 5 μL of Extraction Cocktail (a mixed liquid of 4.8 μL of Extraction Enzyme Dilution Buffer and 0.2 μL of Cell Ectraction Enzyme) was added thereto in accordance with an attached document of PicoPLEX WGA Kit to make a sum of 10 μL. Thereafter, a cytolytic reaction was performed for 10 minutes at 75°C and 4 minutes at 95°C. Next, 5 μL of Pre-Amp Cocktail (a mixed liquid of 4.8 μL of Pre-Amp Reaction Mix and 0.2 μL of Pre-Amp Enzyme) was prepared and was added to the reaction liquid after the cytolysis to make a sum of 15 μL. A reaction corresponding to step (ii) of PicoPLEX WGA Kit was performed as an amplification reaction of DNA. That is, after modifying DNA for 2 minutes at 95°C, a cycle of 15 seconds at 95°C, 50 seconds at 15°C, 40 seconds at 25°C, 30 seconds at 35°C, 40 seconds at 65°C, and 40 seconds at 75°C was performed 30 times.

The obtained amplification product was purified by removing impurities such as a primer component or a buffer component using QIAquick PCR Purification Kit (QIAGEN). The concentration of the amplification product after the purification was measured using Quantus Fluorometer dsDNA System (Promega KK.), and it was confirmed that the amount of amplification product of each cell was within a range of 45 ng to 90 ng (corresponding to 9000 times to 18000 times the amount of chromosomal DNA of one human cell).

[Secondary Amplification Step and First Addition Step (Multiplex PCR)]

16 primer pairs, in which 16 regions on chromosomes were amplified for the purpose of identifying a fetus-derived cell and analyzing heteroploidy of a chromosome, were designed. The 16 regions were regarded as regions having SNP in the inside. The names of genes in which 16 regions are positioned and the base length of the 16 regions are as shown in Table 1.

[Table 1]

|  | Name of gene in which amplification region is positioned | Base length of amplification region |
|---|---|---|
| Chromosome 13 | PABPC1 | 150 bp |
|  | MTRF1 | 180 bp |
|  | PCDH9 | 166 bp |
|  | F10 | 177 bp |
| Chromosome 18 | PIEZO2 | 152 bp |
|  | CDH2 | 166 bp |
|  | MRO | 150 bp |
|  | ALPK2 | 180 bp |
| Chromosome 21 | HUNK | 163 bp |
|  | PRDM15 | 171 bp |
|  | PDE9A | 174 bp |
|  | TSPEAR | 110 bp |

(continued)

|  | Name of gene in which amplification region is positioned | Base length of amplification region |
| --- | --- | --- |
| X chromosome | TBL1X | 104 bp |
|  | CTPS2 | 120 bp |
|  | PIM2 | 164 bp |
| Y chromosome | KDM5D | 143 bp |

Each primer is an oligonucleotide with 37 bases in total which include 17 bases of a base sequence of a first label and 20 bases of a base sequence complementary to a target region, and the base sequence of the first label is positioned at the 5'-terminal. A base sequence of the first label on a forward primer was set to CGCTCTTCCGATCTCTG (SEQ ID No: 1) and a base sequence of the first label on a reverse primer was set to CGCTCTTCCGATCTGAC (SEQ ID No: 2).

All of the primers were mixed with each other such that each final concentration becomes 25 nmol/L to prepare a primer-mixed liquid.

10 ng of a primary amplification product (whole genome amplification product) was used for the secondary amplification step. In multiplex PCR, the reaction was performed using Multiplex PCR Assay Kit (TAKARA BIO INC.). A reaction liquid obtained by mixing 10 ng of a primary amplification product obtained from each cell as a template, 8 $\mu$L of a primer-mixed liquid, 0.125 $\mu$L of Multiplex PCR Mix 1, 12.5 $\mu$L of Multiplex PCR Mix 2, and water was prepared (final amount of liquid being 25 $\mu$L). In the PCR reaction, after modification performed for 60 seconds at 94°C, a cycle of 30 seconds at 94°C, 90 seconds at 60°C, and 30 seconds at 72°C was performed 15 times. The number of cycles of PCR was set based on results obtained by previously performing review through real time PCR.

The obtained amplification product was purified by removing impurities such as a primer component or a buffer component using QIAquick PCR Purification Kit (QIAGEN). The concentration of the amplification product after the purification was measured using Quantus Fluorometer dsDNA System (Promega KK.). It was confirmed that the amount of the amplification product of each cell was within a range of 300 ng to 600 ng (corresponding to 30 times to 60 times the amount of template).

[Tertiary Amplification Step and Second Addition Step (Index Addition PCR)]

The following oligonucleotide was designed as a primer pair used for tertiary amplification.

- An oligonucleotide sequentially having a sequence for binding a flow cell (P5 sequence provided by Illumina, Inc.), an index sequence for identifying a sample, and a sequence annealing to a first label, from the 5'-terminal. D501 provided by Illumina, Inc. was used as an index sequence.
- An oligonucleotide sequentially having a sequence for binding a flow cell (P7 sequence provided by Illumina, Inc.), an index sequence for identifying a sample, and a sequence annealing to a first label, from the 5'-terminal. Any of D701 to D711 provided by Illumina, Inc. was used for each of 11 cells as the index sequence.

5 ng of a labeled secondary amplification product was used for the tertiary amplification step. In PCR, the reaction was performed using Multiplex PCR Assay Kit (TAKARA BIO INC.). A reaction liquid obtained by mixing 5 ng of a secondary amplification product as a template, 1 $\mu$L of each primer (1.25 $\mu$mol/L), 0.125 $\mu$L of Multiplex PCR Mix 1, 12.5 $\mu$L of Multiplex PCR Mix 2, and water was prepared (final amount of liquid being 25 $\mu$L). In the PCR reaction, after modification performed for 3 minutes at 94°C, a cycle of 45 seconds at 94°C, 60 seconds at 50°C, and 30 seconds at 72°C was performed 5 times. Thereafter, a cycle of 45 seconds at 94°C, 60 seconds at 55°C, and 30 seconds at 72°C was performed 11 times. The number of cycles of PCR was set based on results obtained by previously performing review through real time PCR.

The obtained amplification product was purified using AMPure XP Kit (BECMAN COULTER). The concentration of the amplification product after the purification was measured using BioAnalyzer (Agilent Technologies). Quantitative determination was performed as more accurate quantitative determination of the amplification product using KAPA Library Quantification Kits (NIPPON Genetics Co, Ltd). It was confirmed that the amount of amplification product of each cell was within a range of 50 ng to 75 ng (corresponding to 10 times to 15 times the amount of template).

A summary of the amount of amplification product and the amplification factor of 11 cells in each amplification step is as in Table 2.

[Table 2]

|  | Primary amplification step | Secondary amplification step | Tertiary amplification step |
|---|---|---|---|
| Amount of amplification product | 45 ng to 90 ng | 300 ng to 600 ng | 50 ng to 75 ng |
| Amplification factor | 9000 times to 18000 times | 30 times to 60 times | 10 times to 15 times |

[Sequence Analysis Step]

tertiary amplification products derived from 11 cells were mixed with each other and a sequencing was performed using MiSeq (Illumina, Inc.) and MiSeq Reagent Kit v2 300 Cycle (Illumina, Inc.). Mapping of the obtained FastQ file was performed on a human reference genome (hg19) using Burrows-Wheeler Aligner (BWA), the information of genetic polymorphism was extracted using SAMtools, and the number of sequence leads of the amplification regions was calculated using BEDtools to perform analysis.

[Identification of Origin of Cell]

SNP of regions amplified from chromosome 13, chromosome 18, and chromosome 21 was analyzed. As a result, it was confirmed that one cell out of 11 cells had SNP information different from the others. A cell expected to be a white blood cell from the shape of a nucleus was collected from the top of a specimen slide using a micromanipulator and DNA was amplified in the same manner as those of the 11 cells to check SNP. As a result, it was confirmed that SNP of 10 cells except for the above-described one cell and SNP of the cell expected to be a white blood cell were coincident with each other. From the above-described analysis, it was confirmed that the one cell was a fetus-derived nucleated red blood cell and the 10 cells were mother-derived nucleated cells.

[Detection of Presence or Absence of Heteroploidy]

The amount of amplification product in 4 regions of chromosome 21 of a nucleated red blood cell identified as being derived from a fetus was determined using MiSeq. In addition, the amounts of amplification products in 4 regions of chromosomes 21 of 10 nucleated cells identified as being derived from a mother were determined using MiSeq. The proportions of the amounts of amplification products in 4 regions between the fetus-derived cell and each of the mother-derived cells were calculated. As a result, it was assumed that the proportions in all cases were values close to 1:1 and a fetus does not have heteroploidy in chromosome 21.

Similarly, it was assumed that fetuses do not have heteroploidy also in chromosome 13, chromosome 18, an X chromosome, and a Y chromosome.

<Comparative Example 1>

A cell collected from a specimen slide was set as a material. The detection of the presence or absence of heteroploidy of a chromosome was attempted in the same manner as that in Example 1 except that amplification products which were 500,000 times the amount of chromosomal DNA of a human cell were obtained by performing step (i) to step (iii) of PicoPLEX WGA Kit in the primary amplification step. However, the variation between the amounts of amplification products in 16 regions was large and the level of the amplification products was significantly off from a level in which it was possible to detect the presence or absence of heteroploidy.

<Comparative Example 2>

A cell collected from a specimen slide was set as a material. The detection of the presence or absence of heteroploidy of a chromosome was attempted in the same manner as that in Example 1 except that amplification products which were 500 times the amount of a template were obtained by performing 30 cycles of PCR in the secondary amplification step. However, the variation between the amounts of amplification products in 16 regions was large and the level of the amplification products was significantly off from a level in which it was possible to detect the presence or absence of heteroploidy.

The whole disclosure of Japanese Patent Application No. 2014-185060, filed September 11, 2014 is incorporated by reference.

All of the documents, the patent application, and the technical standards disclosed in the present specification are incorporated in the present specification by reference to the same degree as a case where incorporation of individual documents, patent applications, and technical standards by reference is specifically and individually described.

**Claims**

**1.** A method for detecting the presence or absence of heteroploidy of fetal chromosomes, the method comprising:

a primary amplification step of obtaining a primary amplification products by amplifying chromosomal DNA obtained from a biological sample collected from a pregnant mother;
a secondary amplification step of performing multiplex amplification of a plurality of target regions using a plurality of primer pairs to obtain a secondary amplification product using the primary amplification product as a template;
a first addition step of adding a first label, which is oligonucleotide, to both terminals of the secondary amplification product to obtain the labeled secondary amplification product;
a tertiary amplification step of performing amplification using a primer pair annealing to the first label using the labeled secondary amplification product as a template to obtain a tertiary amplification product; and
a sequence analysis step of determining the amplification amount and base sequences of the plurality of target regions from the tertiary amplification product,
wherein the primary amplification step is a step of amplifying the total amount of DNA by 6000 times to 30000 times, and
wherein the secondary amplification step is a step of amplifying the total amount of DNA by 3 times to 150 times.

2.  The method for detecting the presence or absence of heteroploidy of fetal chromosomes according to claim 1,

    wherein the tertiary amplification step is a step of amplifying the total amount of DNA by 6 times to 24 times.

3.  The method for detecting the presence or absence of heteroploidy of fetal chromosomes according to claim 1 or 2,

    wherein the sequence analysis step is performed using a next-generation sequencer.

4.  The method for detecting the presence or absence of heteroploidy of fetal chromosomes according to any one of claims 1 to 3, the method further comprising:

    a second addition step of adding a second label, which is oligonucleotide, to both terminals of the tertiary amplification product to obtain the labeled tertiary amplification product.

5.  The method for detecting the presence or absence of heteroploidy of fetal chromosomes according to claim 4,

    wherein the primer used in the tertiary amplification step has a base sequence of the second label at the 5'-terminal, and
    wherein the second label is added to both terminals of the tertiary amplification product and the labeled tertiary amplification product is obtained by performing the tertiary amplification step which is performed together with the second addition step in this manner.

6.  The method for detecting the presence or absence of heteroploidy of fetal chromosomes according to any one of claims 1 to 5,

    wherein the primer used in the secondary amplification step has a base sequence of the first label at the 5'-terminal, and
    wherein the first label is added to both terminals of the secondary amplification product and the labeled secondary amplification product is obtained by performing the secondary amplification step which is performed together with the first addition step in this manner.

7.  The method for detecting the presence or absence of heteroploidy of fetal chromosomes according to any one of claims 1 to 6,

    wherein the primary amplification step is a step of performing whole genome amplification from the chromosomal DNA.

8.  The method for detecting the presence or absence of heteroploidy of fetal chromosomes according to any one of claims 1 to 7,

    wherein the chromosomal DNA is chromosomal DNA obtained from one cell, and
    wherein the primary amplification step is a step of performing whole genome amplification from one cell.

9.  The method for detecting the presence or absence of heteroploidy of fetal chromosomes according to any one of claims 1 to 8,

    wherein the biological sample is blood, and
    wherein the chromosomal DNA is chromosomal DNA obtained from a cell in blood.

10. The method for detecting the presence or absence of heteroploidy of fetal chromosomes according to claim 9,

    wherein the cell in blood is a nucleated red blood cell.

11. The method for detecting the presence or absence of heteroploidy of fetal chromosomes according to claim 9 or 10,

wherein the cell in blood is a cell isolated through density gradient centrifugation and image analysis.

**EP 3 192 879 A1**

<table>
<tr><td colspan="2" align="center">**INTERNATIONAL SEARCH REPORT**</td><td>International application No.<br>PCT/JP2015/062893</td></tr>
</table>

**A. CLASSIFICATION OF SUBJECT MATTER**

*C12Q1/68*(2006.01)i, *C12N15/09*(2006.01)i

According to International Patent Classification (IPC) or to both national classification and IPC

**B. FIELDS SEARCHED**

Minimum documentation searched (classification system followed by classification symbols)
C12Q1/68, C12N15/09

Documentation searched other than minimum documentation to the extent that such documents are included in the fields searched

| | | | |
|---|---|---|---|
| Jitsuyo Shinan Koho | 1922–1996 | Jitsuyo Shinan Toroku Koho | 1996–2015 |
| Kokai Jitsuyo Shinan Koho | 1971–2015 | Toroku Jitsuyo Shinan Koho | 1994–2015 |

Electronic data base consulted during the international search (name of data base and, where practicable, search terms used)
CAplus/MEDLINE/EMBASE/BIOSIS(STN), JSTPlus/JMEDPlus/JST7580(JDreamIII), DWPI(Thomson Innovation)

**C. DOCUMENTS CONSIDERED TO BE RELEVANT**

| Category* | Citation of document, with indication, where appropriate, of the relevant passages | Relevant to claim No. |
|---|---|---|
| A | WO 2013/057568 A1 (MULTIPLICOM NV),<br>25 April 2013 (25.04.2013),<br>& JP 2014-530024 A & EP 2768978 A1<br>& US 2014/0272979 A1 | 1-11 |
| A | WO 2013/075100 A1 (CELLSCAPE CORP.),<br>23 May 2013 (23.05.2013),<br>& JP 2014-533509 A & EP 2780465 A1<br>& US 2013/0130265 A1 | 1-11 |
| A | JP 2014-507141 A (Natera, Inc.),<br>27 March 2014 (27.03.2014),<br>& WO 2012/108920 A1 & EP 2572003 A1<br>& US 2011/0288780 A1 | 1-11 |

[X] Further documents are listed in the continuation of Box C.   [ ] See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |
| Date of the actual completion of the international search<br>12 June 2015 (12.06.15) | Date of mailing of the international search report<br>23 June 2015 (23.06.15) |
| Name and mailing address of the ISA/<br>Japan Patent Office<br>3-4-3,Kasumigaseki,Chiyoda-ku,<br>Tokyo 100-8915,Japan | Authorized officer<br><br>Telephone No. |

Form PCT/ISA/210 (second sheet) (July 2009)

**INTERNATIONAL SEARCH REPORT**

| International application No. |
|---|
| PCT/JP2015/062893 |

C (Continuation).   DOCUMENTS CONSIDERED TO BE RELEVANT

| Category* | Citation of document, with indication, where appropriate, of the relevant passages | Relevant to claim No. |
|---|---|---|
| A | WO 2014/099919 A2  (ARIOSA DIAGNOSTICS, INC.), 26 June 2014 (26.06.2014), & EP 2668605 A2         & US 2013/0178371 A1 | 1-11 |
| A | WO 2007/147073 A2  (LIVING MICROSYSTEMS, INC.), 21 December 2007 (21.12.2007), & EP 2029778 A2         & US 2008/0050739 A1 | 1-11 |
| A | LIAO GJ., et al., Targeted massively parallel sequencing of maternal plasma DNA permits efficient and unbiased detection of fetal alleles, Clin. Chem., 2011, vol.57, no.1, p.92-101 | 1-11 |
| A | ZIMMERMANN B., et al., Noninvasive prenatal aneuploidy testing of chromosomes 13, 18, 21, X, and Y, using targeted sequencing of polymorphic loci, Prenat. Diagn., 2012, vol.32, no.13, p.1233-1241 | 1-11 |
| A | Akihiko SEKIZAWA, "Botaiketsu o Mochiita Taiji Idenshi Shindan no Genjo", Symposium on Japan Society of Perinatal and Neonatal Medicine Shorokushu, 01 September 2014 (01.09.2014), no.32, pages 51 to 55 | 1-11 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 4404553 B
- JP 2014502845 A
- JP 2004511215 A
- JP 6327476 A
- JP H6327476 A
- JP 2006505268 A
- WO 2012166425 A
- WO 2012023298 A
- JP 2014185060 A